# EUROPÄISCHE PATENTANMELDUNG

(11) **EP 0 926 122 A1**
(43) Veröffentlichungstag der Anmeldung: **30.06.1999**
(21) Anmeldenummer: 98123638.3
(22) Anmeldetag: 10.12.1998
(51) Int. Cl.: C07C 45/69, C07C 49/413

(54) **Verfahren zur Herstellung substituierter Cycloketone**

(30) Priorität: 23.12.1997 DE 19757543; 23.11.1998 DE 19853862
(71) Anmelder: HAARMANN & REIMER GMBH, D-37601 Holzminden (DE)
(72) Erfinder: Krempel, Alfred, 37603 Holzminden (DE); Koch, Oskar Dr., 37079 Göttingen (DE); Erfurt, Harry, 37170 Schönhagen (DE)
(74) Vertreter: Mann, Volker, Dr.

(57) **Zusammenfassung**

Die Erfindung betrifft ein neues Verfahren zur Herstellung Hydroxyalkyl- und Acyloxyalkyl-substituierter Cycloketone durch radikalische Addition von Hydroxy- bzw. Acyloxyalkenen an cyclische Ketone.

## Beschreibung

Die vorliegende Erfindung betrifft ein neues Verfahren zur Herstellung Hydroxyalkyl- und Acyloxyalkyl-substituierter Cycloketone durch radikalische Addition von Hydroxy- bzw. Acyloxyalkenen an cyclische Ketone.

Die Reaktion an sich ist bereits aus der DE-AS 21 36 496 bekannt; sie wird in der Regel in Gegenwart organischer Peroxide (z.B. Di-tert.-butyl-peroxid) durchgeführt. Dabei wird z.B. Cyclododecanon im Überschuß (mindestens 4 Mole pro Mol Alken) vorgelegt und innerhalb von 6 Stunden ein Gemisch aus Allylalkohol und Di-tert.-butyl-peroxid (10-20 Mol-%, bezogen auf Alken) bei 140-150°C zugegeben.

Obwohl derartige Radikalreaktionen prinzipiell in wenigen Minuten ablaufen, wird bei dieser diskontinuierlichen Verfahrensweise der Allylalkohol sehr langsam zugegeben, um Nebenreaktionen, die zu polymerem Material führen, möglichst zu unterdrücken.

Trotz dieser langsamen Zugabe entstehen erhebliche Mengen an polymeren Nebenprodukten. Ein weiterer Nachteil ist die aufgrund der langen Dosierzeit bedingte schlechte Raum/Zeit-Ausbeute.

Aufgabe der hier vorliegenden Erfindung war es somit, diese Nachteile weitgehend zu vermeiden.

Die obengenannte Aufgabe konnte nunmehr dadurch gelöst werden, daß man die radikalische Addition von Oxoalkenen an Cycloketone in Gegenwart organischer Peroxide als Radikalstarter in einem kontinuierlichen Prozeß durchführt.

Überraschenderweise wurde festgestellt, daß durch eine kontinuierliche Verfahrensführung eine Verweilzeit im Reaktor von vorzugsweise nur 30 bis 80 Minuten ausreicht, um eine hohe Raum/Zeit-Ausbeute mit nur geringen Anteilen an polymeren Nebenprodukten zu erhalten.

Gegenstand der Erfindung ist ein Verfahren zur Herstellung von Verbindungen der Formel 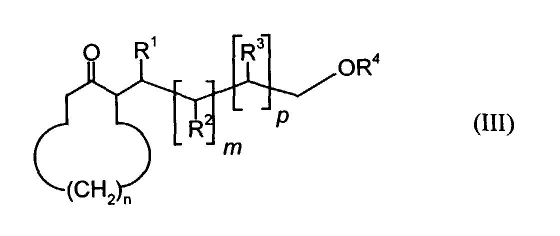 worin
- R¹ bis R³: unabhängig voneinander Wasserstoff, Methyl oder Ethyl,
- R⁴: Wasserstoff oder C₁-C₈-Acyl,
- n: eine ganze Zahl von 6 bis 10 und
- m und p: unabhängig voneinander Null oder 1 bedeuten,
durch Umsetzung eines cyclischen Ketons der Formel 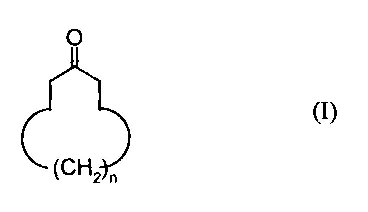 worin
- n: die oben angegebene Bedeutung besitzt,
mit einem Alkenol oder Alkenolester der Formel 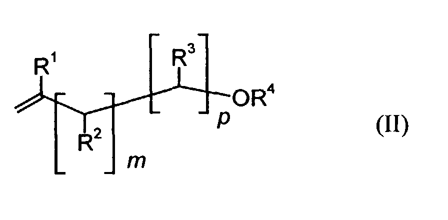 worin
R¹ - R⁴, m, n und p die oben angegebenen Bedeutungen besitzen,
in Gegenwart eines Radikalstarters, dadurch gekennzeichnet, daß man das Verfahren kontinuierlich durchführt.

Als erfindungsgemäße substituierte Cycloketone seien beispielsweise genannt:

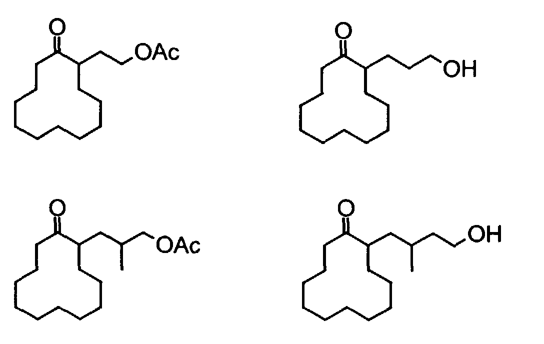

Cyclische Ketone für das erfindungsgemäße Verfahren können beispielsweise sein:

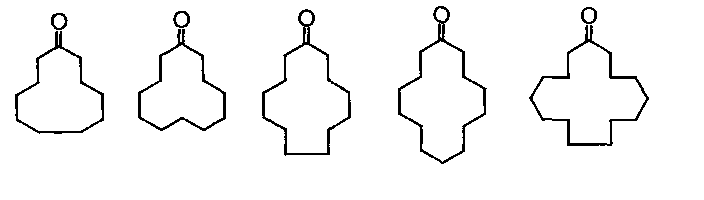

Alkenole für das erfindungsgemäße Verfahren können beispielsweise sein: Vinylacetat, 2-Methyl-propen-2-ol-1, Isopropenylacetat, Buten-3-ol-1, 2-Methyl-buten-3-ol-1, Allylalkohol, Allylacetat, 3-Methyl-buten-3-ol-1.

Radikalstarter für das erfindungsgemäße Verfahren können beispielsweise sein: Cumolhydroperoxid, Peroxyester, wie z.B. tert.-butyl-peroxypivalat, Peroxydicarbonate, wie z.B. Di-(2-Ethylhexyl)-peroxy-dicarbonat, und Azoverbindungen, wie z.B. 2,2'-Azo-bis-(4-methoxy-2,4-dimethylvaleronitril).

Die daraus entstehenden Hydroxyalkylketone III können mit Hilfe von sauren Katalysatoren zu cyclischen Enolethern, wie sie in der DE-AS 21 36 496 bereits beschrieben sind umgesetzt werden. Diese bicyclischen Enolether sind wiederum sehr gute Ausgangsmaterialien zur Herstellung von Riechstoffen.

Die Temperatur bei dem erfindungsgemäßen Verfahren kann, je nach eingesetztem Radikalstarter, 80 bis 200°C betragen; bei Verwendung des besonders bevorzugten Di-tert.-butyl-peroxids liegt die bevorzugte Reaktionstemperatur 130 bis 180°C.

Es ist vorteilhaft, den Druckbereich so zu wählen, daß eine homogene Reaktion ablaufen kann, um gegebenenfalls auch leichtsiedende Ausgangskomponenten, wie z.B. Allylalkohol, einsetzen zu können, deren Siedepunkt unter der ihr den Radikalstarter notwendigen Reaktiontemperatur liegt. Ein bevorzugter Druckbereich liegt z.B. bei 3 bis 50 bar. Besonders bevorzugt wird bei einem Druck von 10 bis 30 bar gearbeitet.

Ein weiterer Vorteil dieses kontinuierlichen Verfahrens ist, daß die überschüssigen Ausgangskomponenten durch fortlaufendes Abdestillieren vom Produkt wieder in den Reaktionsprozeß zurückgeführt werden können.

Ferner ist bei diesem Vorgehen die Möglichkeit gegeben, die Edukte bzw. den Katalysator an verschiedenen Positionen entlang des Reaktionsrohres zuzugeben, um damit je nach Empfindlichkeit und Reaktionsfähigkeit der Einsatzmaterialien zu variieren oder eine Komponente stets in beträchtlichem Überschuß zu halten. Dies kann Neben- und Folgereaktionen reduzieren.

Die kontinuierliche Verfahrensweise ist mit einer deutlichen Steigerung der Raum/Zeit-Ausbeute verbunden; sie ist dem herkömmlichen diskontinuierlichen Verfahren daher weit überlegen.

### Beispiel 1

### Herstellung von 2-(3-Hydroxypropyl)-cyclododecanon

Aus einem auf 100°C thermostatisierten Doppelwandgefäß aus Glas mit Magnetrührer, das als Vorratsbehälter für das Cyclododecanon dient, werden 400 g/h auf eine Mischersäule, die auf 100°C temperiert ist, gepumpt. In die Zuleitung der Mischersäule werden 21.4 g/h eines Gemischs aus Allylalkohol (12.2 g/h) und Di-tert.-butylperoxid (9.2 g/h) fortlaufend dosiert.

Nach Verlassen der Mischersäule fließt das Reaktionsgemisch auf eine auf 170°C thermostatisierte 120 m lange Reaktionskapillare mit 1 mm Durchmesser. Bei einer Lineargeschwindigkeit von 15 cm/sec ergibt sich eine mittlere Verweilzeit von ca. 40 Minuten. Vom Ausgang dieser Kapillare fließt das Reaktionsgemisch auf eine auf 100°C beheizte Rücklaufkapillare in das Vorratsgefäß zurück. Der Innendruck in der Reaktionskapillare wird durch ein Überdruckventil auf ca. 20 bar gehalten.

In den ersten drei Stunden wird dieses Reaktionsgemisch kontinuierlich umgepumpt, wobei die oben angeführte Dosierung des Allylalkohol/Di-tert.-butylperoxid-Gemischs beibehalten wird. Hiernach hat sich ein Gehalt an Hydroxypropylcyclododecanon von ca. 14 Gew.-% in dem Reaktionsgemisch gebildet.

Nach diesen drei Stunden werden dann kontinuierlich über ein Nadelventil am Ende der Reaktionskapillare ca. 100 g/h Produkt ausgeschleust, wobei man gleichzeitig über ein weiteres T-Stück 100 g/h frisches Cyclododecanon in den Zulauf zur Mischersäule einspeist und außerdem die Dosiergeschwindigkeit des Allylalkohol/Di-tert.-butylperoxid-Gemischs auf 16.4 g/h (9.4 bzw. 7 g/h) reduziert. Nach 7 Stunden werden Pumpen und Entnahme gestoppt.

### Bilanz nach 10 h Gesamtreaktionszeit:

| | |
|---|---|
| Einsatz: | 1 100 g (6.04 Mol) Cyclododecanon |
| | 102 g (1.76 Mol) Allylalkohol |
| | 77 g (0.53 Mol) Di-tert.-butylperoxid |
| Gesamt: | 1 279 g Reaktionsgemisch |
| Produktinhalt: | ca. 14 Gew.-% = 179 g Produkt |

Die Ausbeute, bezogen auf Allylalkohol, beträgt 42 %.

### Vergleich (Batch-Verfahren)

Man legt 1 179 g Cyclododecanon vor, heizt auf 150°C auf und dosiert unter Rühren innerhalb von 8 Stunden gleichzeitig 69 g Allylalkohol und 31 g Di-tert.-butylperoxid aus getrennten Vorlagen. Nach Dosierende wird bei der genannten Temperatur noch eine halbe Stunden nachgerührt. Man kühlt ab und erhält nach insgesamt 10 Stunden 1 279 g Reaktionsgemisch mit einem Gehalt von 10 % Hydroxypropylcyclododecanon.

### Bilanz nach 10 h Gesamtreaktionszeit:

| | |
|---|---|
| Einsatz: | 1 179 g (6.48 Mol) Cyclododecanon |
| | 69 g (1.19 Mol) Allylalkohol |
| | 31 g (0.21 Mol) Di-tert.-butylperoxid |
| Gesamt: | 1 279 g Reaktionsgemisch |
| Produktinhalt: | ca. 10 Gew.-% = 128 g Produkt. |

Die Ausbeute beträgt, bezogen auf Allylalkohol, 45 %.

Dieser Vergleich belegt, daß das konti-Verfahren nach bereits 10 Stunden Reaktionszeit bei gleicher Gesamteinsatzmenge an Edukten eine um den Faktor 1,4 bessere Produktausbeute ergibt. Weiterhin liegt das Eduktverhältnis Cyclododecanon/Allylalkohol im konti-Verfahren bei 3.4:1 gegenüber 5.5:1 beim Batch-Verfahren, so daß ein deutlich geringerer Überschuß an Cyclododecanon nötig ist. Dies hat u.a. auch zur Folge, daß bei der Aufarbeitung weniger Cyclododecanon abdestilliert werden muß.

### Beispiele 2 bis 6

Die Beispiele wurden in Analogie zu Beispiel 1 durchgeführt:

| **Beispiel** | **Substituiertes Cycloketon** | **Cyclisches Keton** | **Alkenol** | **Radialstarter** | **Ausbeute** |
|---|---|---|---|---|---|
| **2** | 2-(2-Acetoxyethyl)-cyclododecanon | Cyclododecanon | Vinylacetat | Di-tert.-butylperoxid | 50% |
| **3** | 2-(3-Acetoxy-2-methylpropyl)-cyclododecanon | " | Isopropenyl-acetat | " | 50% |
| **4** | 2-(4-Hydroxy-2-methyl-butyl)-cyclododecanon | " | 3-Methyl-buten-3-ol-1 | Azo-bis-isobutyronitril | 30% |
| **5** | 2-(3-Hydroxy-2-methylpropyl)-cycloundecanon | Cycloundecanon | 2-Methylpropen2-ol-1 | tert.-Butyl-peroxypivalat | 35% |
| **6** | 2-(3-Hydroxypropyl)-cyclododecanon | Cyclododecanon | Allylalkohol | Di-(2-Ethylhexyl)-peroxydicarbonat | 35% |

## Patentansprüche

1. Verfahren zur Herstellung von Verbindungen der Formel 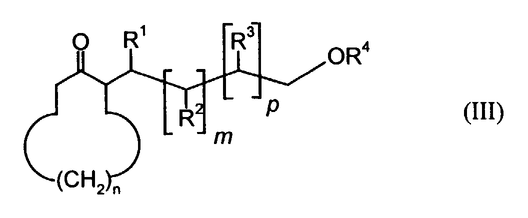 worin
R¹ bis R³ unabhängig voneinander Wasserstoff, Methyl oder Ethyl,
R⁴ Wasserstoff oder C₁-C₈-Acyl,
n eine ganze Zahl von 6 bis 10 und
m und p unabhängig voneinander Null oder 1 bedeuten,
durch Umsetzung eines cyclischen Ketons der Formel 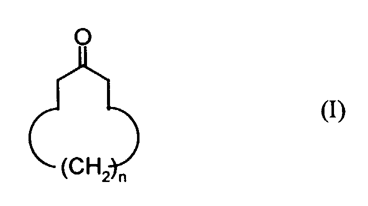 worin
n die oben angegebene Bedeutung besitzt,
mit einem Alkenol oder Alkenolester der Formel 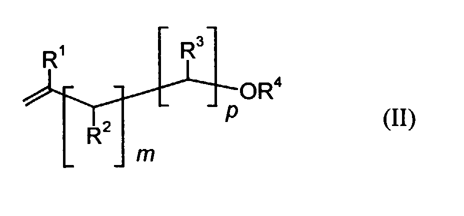 worin
R¹ - R⁴, m, n und p die oben angegebenen Bedeutungen besitzen,
in Gegenwart eines Radikalstarters, dadurch gekennzeichnet, daß man das Verfahren kontinuierlich durchführt.

2. Verfahren nach Anspruch 1, wonach die mittlere Verweilzeit der Reaktanden im Reaktor 30 bis 80 Minuten beträgt.

3. Verfahren nach Anspruch 1, dadurch gekennzeichnet, daß man die Reaktion bei einer Temperatur von 80 bis 200°C durchführt.

4. Verfahren nach Anspruch 1, dadurch gekennzeichnet, daß man die Reaktion unter einem Druck von 3 bis 50 bar durchführt.

5. Verfahren nach Anspruch 4, dadurch gekennzeichnet, daß man die Reaktion unter Druck in einem Rohrreaktor durchführt.

6. Verfahren nach Anspruch 1, dadurch gekennzeichnet, daß man nach Erhalt des Reaktionsgemisches die Verbindung III abtrennt und die nicht umgesetzten Ausgangsmaterialien in den Reaktor zurückführt.
